# FASCICULE DE BREVET EUROPEEN

(11) **EP 1 087 707 B1**
(45) Date de publication et mention de la délivrance du brevet: **07.09.2005**
(21) Numéro de dépôt: 99925125.9
(22) Date de dépôt: 17.06.1999
(51) Int. Cl.: A61B 17/32, A61M 1/00

(54) **PIECE A MAIN DE COMMANDE PNEUMATIQUE D'UN APPAREIL CHIRURGICAL**
HANDSTÜCK ZUR PNEUMATISCHEN STEUERUNG EINER CHIRURGISCHEN VORRICHTUNG
HANDPIECE FOR PNEUMATIC CONTROL OF A SURGICAL APPLIANCE

(30) Priorité: 17.06.1998 FR 9807732
(43) Date de publication de la demande: 04.04.2001
(73) Titulaire: Eschmann Holdings Limited, West Sussex BN15 8TJ (GB)
(72) Inventeur: GONON, Bertrand, F-69002 Lyon (FR)
(74) Mandataire: Metz, Paul
(86) Numéro de dépôt international: PCT/FR1999/001461
(87) Numéro de publication internationale: WO 1999/065407

(56) Documents cités:
- FR-A- 1 378 042
- US-A- 4 205 677
- US-A- 4 299 221
- US-A- 4 655 197

## Description

La présente invention concerne une pièce à main à usage chirurgical ou médical à fonction supplémentaire de commande pour au moins un appareil lié à l'intervention. L'invention se rapporte également au procédé de commande associé.

Cette pièce à main comporte une fonction de commande ou de télécommande par la modification d'un débit ou d'une pression ou d'une dépression et plus particulièrement par une dépression car elle se trouve reliée dans une de ses variantes à une source d'aspiration par au moins un conduit associé à au moins un capteur ou détecteur de commande de fonctionnement.

La pièce à main selon l'invention est notamment, mais non exclusivement, du type de celles connues pour des travaux de dissection mais aussi de lavage-rinçage dans les domaines chirurgicaux et médicaux. Elle est raccordée à cet effet par au moins un conduit à une source de liquide stérile distribué sous haute pression ou sous débit. Elle se trouve traversée de part en part par un tube de jet haute pression ou de débit relié à un générateur ou à un distributeur de liquide stérile. L'exploitation s'effectue à partir de capteurs ou de détecteurs appropriés sensibles à une pression, à une dépression ou à un débit.

On connaît également des pièces à main où le tube de jet haute pression est couplé à un tube d'aspiration. La commande qui permet d'alterner ou d'additionner les fonctions de découpe et/ou d'aspiration se fait généralement en modifiant un paramètre externe à la pièce à main. L'opérateur doit par conséquent relâcher son attention pour manipuler un bouton ou donner un ordre qui ne peut être exécuté en temps réel. Il s'ensuit au mieux un ralentissement de l'opération ou un manque de précision pendant la formulation de l'ordre et dans certains cas différents défauts et maladresse de manipulation.

La pièce à main à fonction de commande ou de télécommande selon l'invention est particulièrement destinée à être utilisée en chirurgie. Elle permet en effet une dissection précise et efficace grâce à la possibilité pour le chirurgien de commander alternativement ou simultanément du bout des doigts et sans effort(s) les fonctions de jet haute pression et d'aspiration ou de nettoyage et d'aspiration, et l'intensité du jet sans relâcher son attention mais aussi d'autre(s) appareil(s).

Une telle pièce à main à fonction de télécommande peut être d'un autre type, utiliser une autre énergie pour la dissection ou le lavage, ou servir à réaliser d'autres fonctions utiles par exemple en médecine ou en chirurgie.

La pièce à main selon l'invention peut également trouver une application intéressante en micromécanique ou dans tout autre domaine où une découpe de qualité est souhaitée.

La commande en pression du jet haute pression ou celle du débit ou de toute autre énergie utilisée dans les applications chirurgicales ou médicales est généralement effectuée par un ou plusieurs interrupteur(s) électriques situé(s) sur la pièce à main et relié(s) par une ou plusieurs liaison(s) filaire(s).

Outre la commande à partir d'interrupteur(s) électrique(s), on connaît des commandes manuelles et mécaniques directes dans lesquelles des pistons poussoirs à rappel élastique vers le haut, à la manière d'un instrument de musique à vent, agissent directement sur les conduits et les liaisons transportant le liquide stérile afin d'en limiter ou d'en arrêter le débit.

Dans le cas d'une commande par interrupteur(s) électrique(s), les liaisons filaires et l'exploitation des signaux électriques de commande en basse intensité peuvent être entachés de défauts de transmission, de parasitages ou de perturbations diverses liées à l'environnement électrique et radioélectrique du lieu opératoire. Ces perturbations conduisent à des modifications involontaires de fonctionnement de la pièce à main et de son alimentation. Ces défauts représentent des risques pouvant engendrer des conséquences graves.

De plus, la présence et le montage de ces liaisons filaires avec l'appareil représentent une gêne et une perte de temps.

Dans le cas d'une commande manuelle directe par poussoirs mécaniques, d'autres inconvénients apparaissent. Cette technologie nécessite la confection de pièces spéciales de précision de petites dimensions et le montage de celles-ci sur la pièce à main. Un des premiers inconvénients concerne le coût de fabrication et de montage. Il faut citer d'autres inconvénients liés aux obligations de désinfection et de stérilisation de la pièce à main imposant le démontage-montage de toutes ces pièces avec divers risques dont la perte éventuelle de divers éléments composants de petite taille.

US 4 299 221 montre un ensemble de commande de fonctionnement pour pièce à main comprenant un conduit de commande agissant sur un élément de pincement d'un tube transportant un fluide d'irrigation en vue de couper ou d'ouvrir l'arrivée du fluide d'irrigation.

La présente invention a pour but de remédier à ces inconvénients en proposant un ensemble de commande de fonctionnement pour pièce à main selon la revendication 1 et un procédé de commande selon la revendication 21.

Les discontinuités ou variations pneumatiques engendrées par l'obturation ou la libération momentanée de cet orifice ou par une restriction résultant d'une déformation plastique ou élastique du conduit ouvert ou fermé par l'opérateur sont exploitées en commande au niveau de l'appareil auquel est branchée la pièce à main considérée ou de tout autre appareil lié à l'intervention.

Selon une caractéristique essentielle, d'un des types de pièce à main selon l'invention, elle comprend, en plus du tube de jet haute pression ou du tube délivrant un débit de liquide stérile, un ou des orifice(s) relié(s) par un ou des conduit(s) à une source de pression ou de dépression existant dans les lieux opératoires via des capteurs ou des détecteurs chargés d'informer l'unité centrale de l'appareil de l'existence d'une commande en vue de son exécution.

Selon un mode de réalisation préféré, ces orifices de commande sont situés de part et d'autre du tube de jet haute pression ou de débit et débouchent symétriquement sur les côtés du corps de la pièce à main.

Selon une variante avantageuse, ces orifices sont situés sur des conformations ergonomiques et anatomiques réalisées sur le corps de la pièce à main au niveau du pouce et de l'index de l'opérateur de manière à permettre une action digitale aisée de commande.

La commande peut s'effectuer par le pouce seul et/ou l'index de l'opérateur qui obture l'entrée du ou des tubes de commande lorsqu'il veut envoyer un ordre de commande ou ouvre momentanément cet orifice resté fermé. Cette commande peut s'effectuer simplement et facilement et sans perturber l'attention de l'opérateur. Elle se traduit par une discontinuité de pression ou de dépression et donc de débit d'air. Les capteurs ou les détecteurs fournissent l'information à l'unité centrale de l'appareil qui l'interprète et l'exécute.

Un moyen équivalent consiste à faire passer dans ces zones ergonomiques au moins un tronçon de conduit déformable ouvert ou fermé en extrémité que l'opérateur pourra aisément écraser par la pression de l'un de ses doigts ou engendrer une fuite momentanée et donner naissance ainsi à une discontinuité de pression ou de dépression interprétée comme une commande. Il peut s'agir du conduit d'aspiration ou d'évacuation qui serait obturé pendant un court instant.

La commande ou télécommande directe par la main de l'opérateur permet une plus grande précision et rapidité dans le travail effectué.

L'utilisation d'une commande pneumatique est simple et propre et insensible aux parasites électriques et radioélectriques existant nombreux dans l'environnement d'un bloc opératoire.

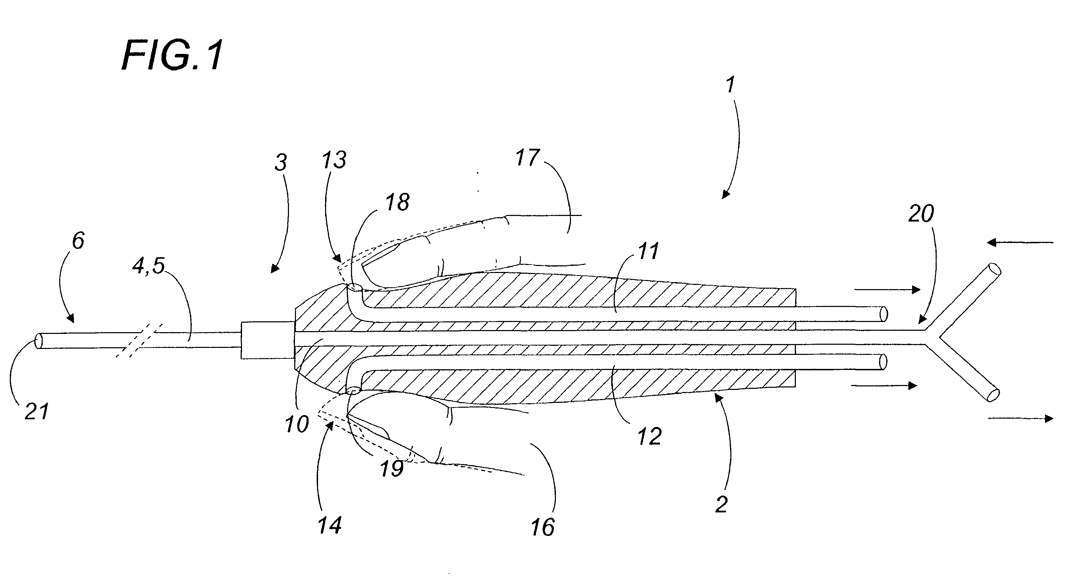

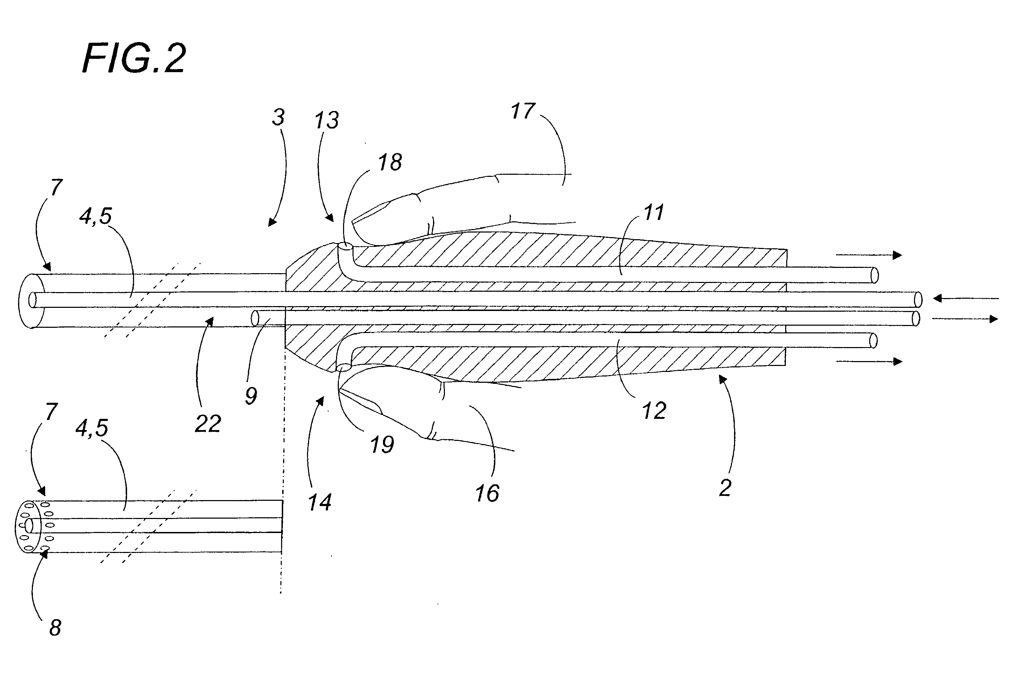

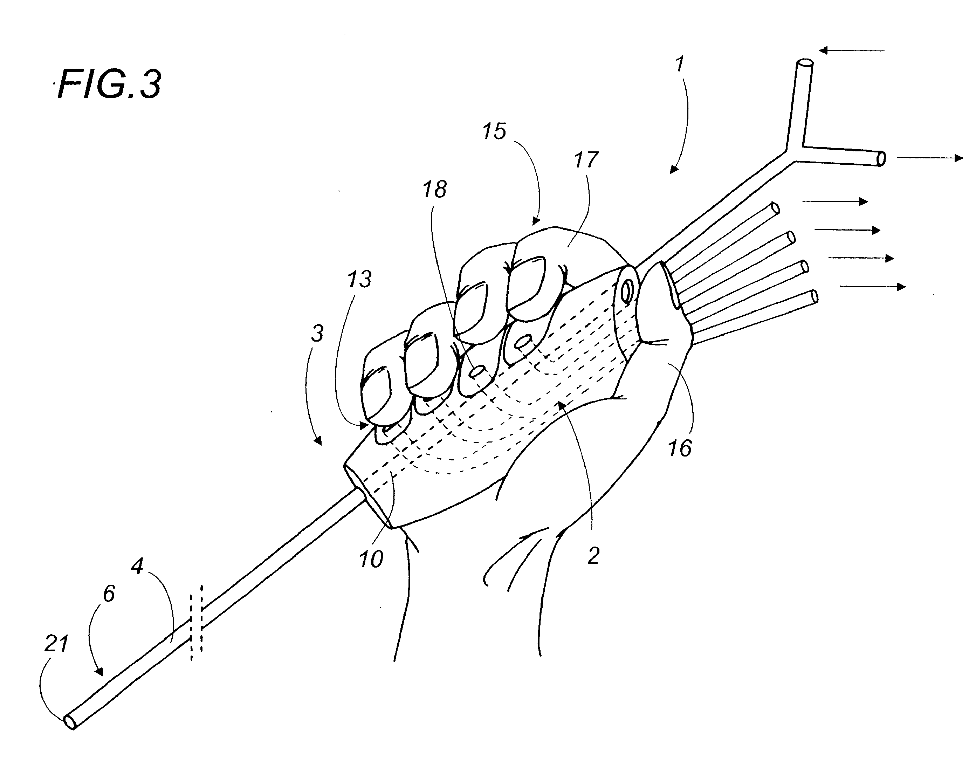

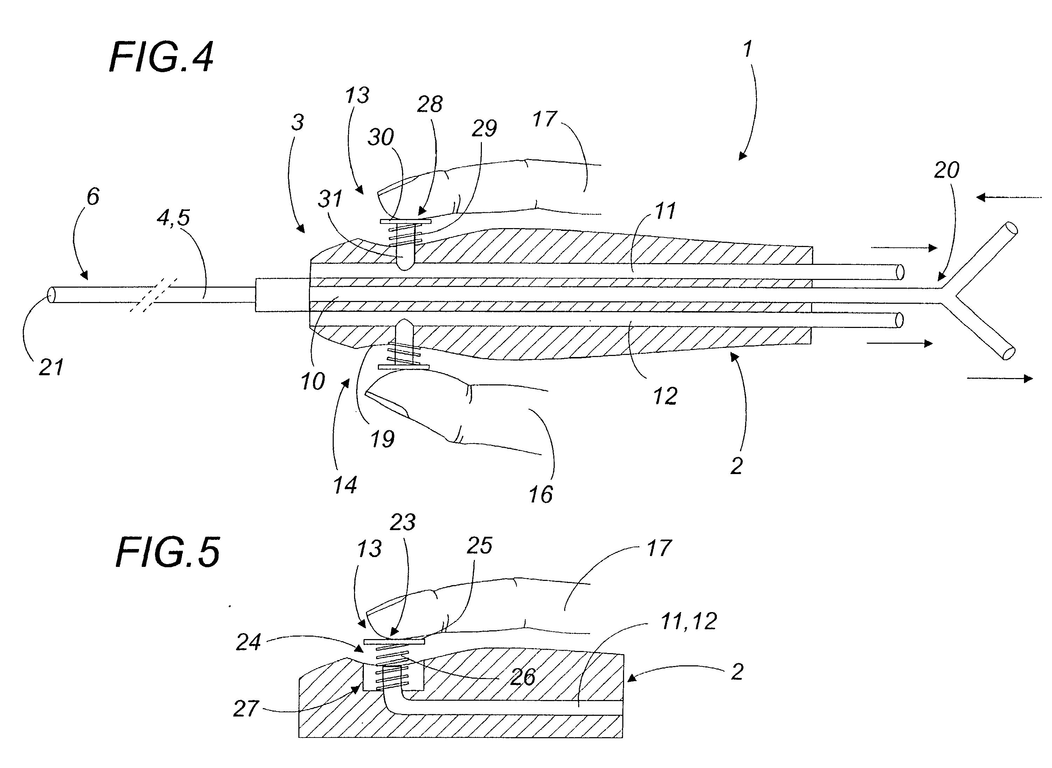

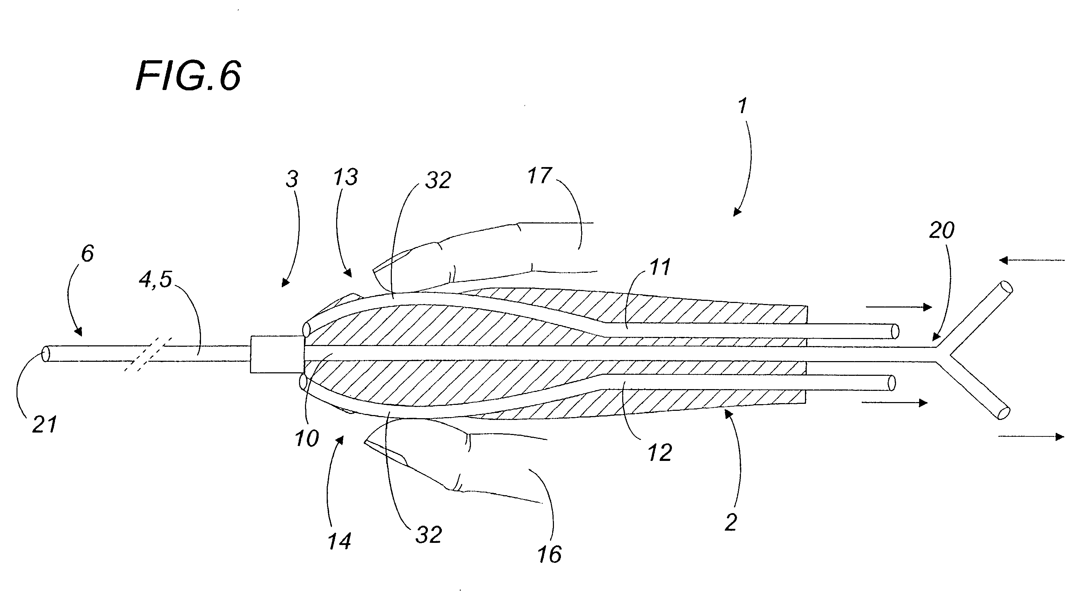

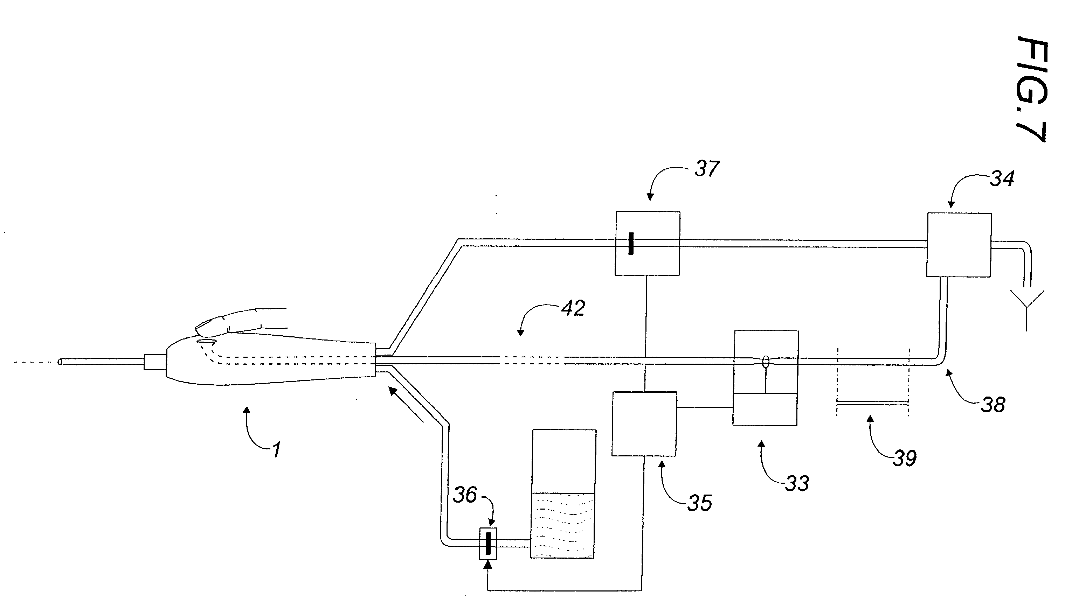

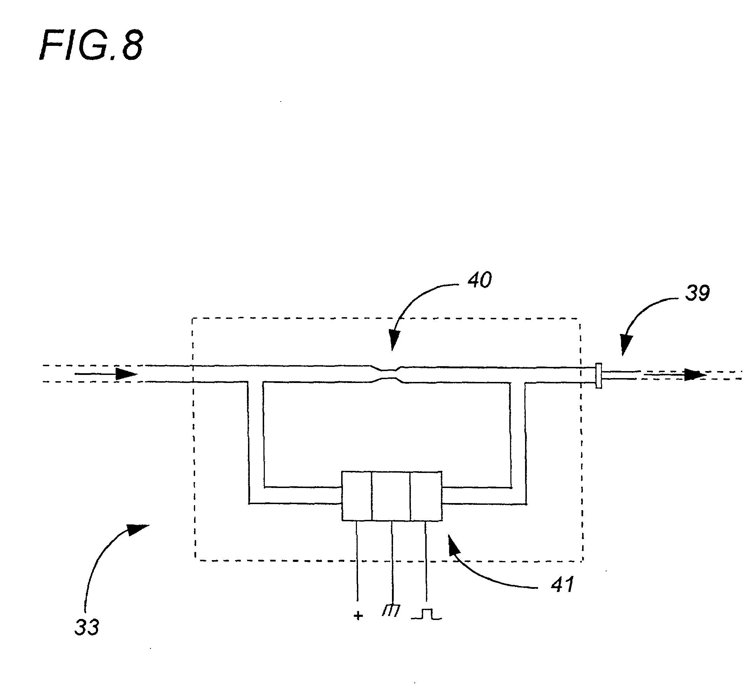

L'invention sera mieux comprise à la lecture de la description qui suit effectuée d'après un mode d'exécution préféré et non limitatif en référence aux dessins dans lesquels :
. la figure 1 représente une vue d'ensemble en coupe longitudinale d'une pièce à main à commande pneumatique selon l'invention avec un tube commun de jet haute pression et deux conduits de commande ;
. la figure 2 représente une vue d'ensemble en coupe longitudinale d'une pièce à main de lavage-rinçage à commande pneumatique selon l'invention avec un tube pour le jet de lavage et un tube parallèle d'aspiration ;
. la figure 3 représente une vue d'ensemble en coupe longitudinale d'une pièce à main d'oellioscopie à commande pneumatique selon l'invention avec une pluralité de conduits de commande pneumatique et les orifices correspondants ;
. la figure 4 est une vue d'ensemble en coupe longitudinale montrant une variante à boutons poussoirs ;
. la figure 5 est une vue partielle en coupe longitudinale montrant un exemple d'obturation du conduit de commande par une pièce d'obturation ;
. la figure 6 est une vue d'ensemble en coupe longitudinale montrant une variante à écrasement du conduit de commande pneumatique ;
. la figure 7 est une vue schématique d'un exemple de montage dans lequel se trouve la pièce à main à commande pneumatique selon l'invention ;
. la figure 8 est une vue en coupe d'un exemple d'un détecteur de discontinuité pneumatique.

La présente invention procède de l'idée générale inventive qui consiste à exploiter une discontinuité pneumatique et plus généralement une variation de pression, de dépression ou de débit engendrée par une pression digitale exercée par la main de l'opérateur au cours de son travail chirurgical ou médical à l'aide d'une pièce à main, par exemple d'aquadissection ou de lavage-rinçage, pour commander à distance le fonctionnement d'un ou de plusieurs appareils alimentant cette pièce à main en fluide et plus généralement d'un ou de plusieurs appareils liés à l'intervention.

L'invention concerne aussi le procédé qui consiste à utiliser au moins une liaison pneumatique reliée à une source de pression ou de dépression débouchant à l'extérieur du corps d'une pièce à main chirurgicale ou médicale par un orifice et à obturer cet orifice directement ou indirectement par une action digitale exercée par un doigt de l'utilisateur ou à agir en modification de section d'un tronçon déformable de ce conduit de commande, pour donner naissance à des signaux pneumatiques utilisés pour la commande ou la télécommande du fonctionnement d'un ou de plusieurs appareils alimentant cette pièce à main ou liés à l'intervention réalisée avec la pièce à main après une détection appropriée par un détecteur, et à exploiter ces signaux après traitement pour commander ce ou ces appareils.

On a représenté sur les figures un exemple particulier de pièce à main. Cet exemple sera décrit ci-après sans pour autant le considérer comme limitatif.

Comme représentée sur les figures, la pièce à main 1, utilisée comme exemple descriptif, est composée d'un corps 2 et d'une tête 3. Un tube 4 de jet haute pression ou un tube 5 de jet de lavage délivrant le liquide stérile de travail sur le champ opératoire traverse la pièce à main 1 de part en part et débouche par exemple au-delà de la tête 3 de la pièce à main 1 par une saillie 6 ou à l'intérieur d'un manchon d'extrémité 7 simple ou à zone annulaire 8 de perforations (figure 2). Ce tube 4 ou 5 est relié à un appareil générateur ou de distribution de liquide stérile sous haute pression ou sous débit comportant un circuit de commande permettant de recevoir et d'exécuter les signaux de commande provenant d'une commande pneumatique.

Le tube 4 du jet haute pression et le tube 5 du jet de lavage sont souvent doublés d'un conduit d'aspiration 9 relié à une source de dépression par exemple au réseau de vide du bloc opératoire. Le conduit d'aspiration 9 de la pièce à main de lavage-rinçage est naturellement de plus grosse section que celui de la pièce à main de haute pression. Ces conduits sont destinés à aspirer et à évacuer le liquide de travail avec les résidus, débris, déchets, éclats ou fragments ou autres provenant du travail de dissection, de raclage, de désagrégation par le jet haute pression ou entraînés par le lavage ou le rinçage.

Il existe aussi des pièces à main à conduit commun 10 de jet liquide stérile de travail et d'aspiration comme représenté sur les figures 1, 4 et 6. Pour ces pièces à main, le fonctionnement entre ces deux fonctions doit être séquentiel.

La présente invention propose d'ajouter au moins un, mais de préférence deux, conduits de commande pneumatique 11 et 12 intégrés dans la pièce à main 1. Ces conduits de commande 11, 12 sont de préférence souples. Ils entrent par l'arrière de la pièce à main de part et d'autre du tube 4 de jet haute pression ou du tube 5 de lavage et débouchent ou passent en saillie par exemple symétriquement sur les côtés du corps 2 de la pièce à main 1 au niveau de deux conformations ergonomiques et anatomiques correspondantes 13 et 14 réalisées de part et d'autre du corps 2 de la pièce à main, situées au niveau de la zone de maintien de la pièce par la main 15 de l'opérateur, plus particulièrement au niveau du pouce 16 et de l'index 17 de l'opérateur (figure 1).

Les conduits 11 et 12 de commande pneumatique sont reliés à une source de pression ou de dépression via des capteurs ou détecteurs appropriés (par exemple du type de ceux représentés sur les figures 7 et 8) par exemple des capteurs de débit ou des détecteurs de discontinuités de pression ou de dépression. On peut par exemple relier ces conduits 11 et 12 de commande au réseau de vide dans les blocs opératoires des hôpitaux ou à une quelconque pompe à vide ou à un compresseur. Les capteurs ou détecteurs sont aptes à transformer la discontinuité ou plus généralement la variation de pression ou de dépression ou la variation de débit de fluide dans ces conduits de commande en un signal électrique et être montés dans un circuit électronique pour renseigner l'unité centrale de l'appareil distributeur du liquide stérile de travail qu'une commande est à exécuter.

Une des techniques de détection consiste à détecter une discontinuité ou variation de pression ou de dépression ou de débit dans le conduit de commande provoquée par l'obturation volontaire de l'orifice d'extrémité ou la modification volontaire de la section du conduit souple de commande directement ou indirectement par le doigt de l'utilisateur. Cette discontinuité ou variation de pression ou de dépression ou de débit est transmise à l'unité centrale, contrôlant par exemple l'appareil de génération ou de distribution du liquide stérile de travail, pour la renseigner sur la commande et déclencher la modification de fonctionnement correspondante.

Lorsque l'opérateur tient la pièce en main, il peut dégager ou obturer directement ou indirectement les orifices des conduits de commande 11 et 12, avec son pouce 16 et/ou son index 17. Lorsqu'il veut envoyer un ordre, il lui suffit de dégager ou d'obturer le ou les orifice(s) 18 ou 19 du ou des conduits 11 ou 12 de dépression afin de provoquer une discontinuité de pression ou de dépression ou de débit qui sera détectée par le ou les détecteur(s) ou capteur(s) correspondants.

On peut aussi coder le nombre d'impulsions de commande et arriver ainsi à augmenter les possibilités générales en commande.

On peut aussi compter ou décompter le nombre d'impulsions et les arrêter au moment où le nombre d'impulsions a atteint celui demandé.

Un seul conduit de commande ne permet d'agir que sur une seule grandeur par exemple la faire varier en intensité. Il est possible cependant d'élargir la commande en codant le nombre d'impulsions de commande. La présence de deux conduits de commande 11 et 12 reliés à deux capteurs ou détecteurs spécifiques permet d'alterner et/ou d'additionner deux fonctions. On gagne ainsi en précision et rapidité d'exécution. Par une simple action directe ou indirecte du pouce 16 et/ou de l'index 17, l'opérateur peut alternativement ou simultanément solliciter le conduit ou le tube 4 ou 5 de distribution du liquide stérile de travail et commander une autre grandeur par exemple doser l'aspiration.

Si l'unité centrale ou si le ou les détecteur(s) ou le ou les capteur(s) est ou sont relié(s) à d'autres appareils utilisés lors de l'intervention chirurgicale ou plus généralement lors d'une intervention quelconque mettant en oeuvre une pièce à main quelconque, l'opérateur peut par une simple action digitale commander le fonctionnement ou la modification de certains paramètres de ces appareils.

A cet effet, il suffit pour l'opérateur de provoquer au moins une impulsion de commande par une simple action digitale d'obturation au niveau de l'orifice sur la pièce à main communiquant avec le générateur de pression ou de dépression. Bien entendu, on peut envisager une succession d'obturations dont par exemple le nombre correspondrait à un ordre particulier de commande. Cette succession codée peut former un signal reconnu par son individualité et correspondant à une commande particulière.

Le chirurgien peut ainsi, très facilement et sans détourner son attention, avoir accès et commander de nombreux paramètres jouant un rôle pendant l'intervention chirurgicale.

Le système de commande se décomposant en deux temps, d'abord l'envoi d'une succession codée par l'opérateur puis traitement du signal par l'unité centrale et exécution de la commande, présente un avantage supplémentaire.

En effet, l'opérateur n'agit pas directement sur les paramètres de fonctionnement lorsqu'il provoque une ou plusieurs variation(s) de pression ou de dépression. Un système de commande directe de ces grandeurs nécessiterait de la part de l'opérateur une manipulation ou du moins un geste rapide et précis pour obtenir une variation pneumatique quantifiée et reproductible et éventuellement modulée provoquant une variation proportionnelle de la valeur du paramètre.

Une telle manipulation peut être source d'imprécisions ou d'erreurs pouvant conduire à de graves conséquences dans un domaine d'application aussi spécifique que celui de la chirurgie ou celui de la médecine.

Le dispositif de commande selon l'invention est donc plus fiable et plus précis, l'opérateur étant certain d'obtenir l'exécution de la commande qu'il a ainsi initiée.

Les capteurs ou détecteurs sont des éléments sensibles à la pression, à la dépression ou au débit. Ils envoient à l'unité centrale l'information qui sera convertie par exemple en ordre pour l'appareil de génération ou de distribution du liquide stérile de travail, de cesser ou de reprendre la génération du jet liquide ou d'en augmenter ou d'en diminuer l'intensité en pression ou en débit ou de lancer un tir isolé ou une succession de tirs ou de modifier le nombre de tirs d'une succession donnée.

Selon le mode de réalisation, illustré par les figures 1, 4 et 6, les conduits 4 et 5 de jet haute pression et d'aspiration sont communs à l'intérieur du corps 2 de la pièce à main 1 en un tube unique 10 qui présente à l'une de ses extrémités une réunion 20 des deux branches et débouche au niveau de la tête 3 en une extrémité unique 21. Dans cette variante de pièce à main, les fonctions de dissection et d'aspiration sont par conséquent alternées.

Il existe aussi des pièces à main mixtes servant aussi bien de pièces à main de travail avec un jet haute pression que de pièces à main de lavage-rinçage. Dans ces pièces à main mixtes, les commandes sont plus nombreuses. Il suffit de prévoir plusieurs lignes de commande ou différents signaux codés.

Selon un autre mode de réalisation, illustré par la figure 2, les conduits 4 ou 5 de jet de haute pression ou de lavage et d'aspiration 9 restent séparés à l'intérieur du corps 2 de la pièce à main 1 pour déboucher au niveau de la tête 3 en une structure bitube 22.

En raison du débit nécessaire du liquide stérile de lavage, le conduit d'aspiration 9 a un diamètre supérieur au diamètre du tube 5 du jet de lavage. Ce mode de réalisation permet d'utiliser simultanément les fonctions de découpe et d'aspiration ce qui permet à l'opérateur de travailler avec rapidité et précision sans être encombré par les débris engendrés par son travail.

Il y a lieu de mentionner également la variante selon laquelle l'obturation des orifices est réalisée par une pièce intermédiaire 23 mobile ou déformable en rappel élastique de libération c'est-à-dire vers une position d'éloignement de l'orifice 18 ou 19 dont un exemple est représenté sur la figure 5.

Il peut s'agir d'une pastille d'obturation 24 montée sur au moins deux rondelles élastiques ou équivalent ou d'une pièce spéciale formée d'un opercule d'obturation 25 en rappel élastique vers le haut par exemple par un ressort 26 et d'un corps en manchon déformable 27 à face latérale perméable à l'air. On peut citer comme exemple une surface latérale sous la forme d'un soufflet traversé par une multitude de perçages ou ajouré par endroits ou d'une structure d'empilement formée d'une succession de rondelles écartées les unes des autres par des moyens élastiques. Tout moyen semblable ou proche convient ainsi que tout moyen équivalent.

Comme indiqué en début de description, rentrent dans le cadre de l'invention tous les moyens permettant à l'opérateur d'engendrer une discontinuité ou tout au moins une variation de pression, de dépression ou de débit par une action d'un de ses doigts sur une pièce à main par exemple d'applications chirurgicales et/ou médicales.

Ainsi, l'invention prévoit une variante représentée sur la figure 4 à boutons poussoirs tels que 28 montés chacun en rappel élastique vers le haut par exemple par un ressort 29. Ces boutons poussoirs présentent une extrémité supérieure en forme de touche 30 et une extrémité inférieure par exemple conformée en lame 31 non coupante afin de réaliser l'interruption nécessaire du débit.

L'invention prévoit également une variante représentée sur la figure 6 selon laquelle la discontinuité ou la variation de pression ou de dépression est apportée par la déformation élastique ou plastique d'un tronçon déformable 32 du conduit relié à la source de pression ou de dépression. Ce tronçon déformable 32 est disposé partiellement ou totalement en saillie sur une longueur suffisante au niveau de la zone d'emplacement naturel du pouce 16 ou de l'index 17 ou d'un autre doigt en vue d'imprimer manuellement directement ou indirectement une discontinuité ou une variation de pression ou de dépression par l'écrasement partiel ou total du conduit au niveau de ce tronçon. Comme précédemment, cette variation de pression ou de dépression est convertie et exploitée en signal de commande par l'unité centrale de l'appareil délivrant le liquide stérile de travail.

Bien entendu, l'écrasement du conduit peut être réalisé au moyen d'une pièce intermédiaire par exemple transversale et montée en rappel élastique vers le haut de manière à faciliter l'écrasement et à améliorer l'étranglement en diminuant encore la restriction du passage jusqu'à l'obturation totale.

D'autres variantes du type à pincement ou à écrasement direct ou à touche sont possibles.

Il faut indiquer également une catégorie de variantes selon lesquelles la détection ne fonctionne plus selon une interruption ou une réduction du débit, mais de façon inverse. Selon ces variantes, le ou les conduit(s) de commande sont obturé(s) ou fermé(s) en permanence, soit sous pression, soit sous dépression constante mais sans débit. La commande consiste à laisser momentanément échapper le fluide gazeux par exemple de l'air en cas de commande avec pression ou de laisser admettre une quantité d'air par une mise momentanée à l'air dans le cas de la commande avec dépression.

A titre de moyens technologiques, il peut être prévu des clapets inversés dans les deux cas de la commande pneumatique avec pression ou avec dépression. Ces clapets sont prévus à l'extrémité des orifices 18 ou 19 des conduits de commande 11 et 12. Ils se trouvent normalement à l'état fermé en l'absence de commande et s'ouvrent momentanément sur action manuelle digitale de l'opérateur.

La commande au niveau du circuit ne présente pas plus de difficulté. Elle se réalise de façon analogue à partir d'un signal de débit correspondant à l'ouverture.

On mentionne également des pièces de pincement ou d'écrasement contraintes en position d'obturation vers le bas par une force de poussée permanente que l'on supprime, neutralise, compense ou autre momentanément pour initier une commande.

Il en est de même pour les variantes à tronçon déformable 32. Les extrémités des conduits de commande 11 et 12 sont fermées et un dispositif approprié à commande manuelle provoque une fuite ou une mise à l'air momentanée pour initier la commande.

La détection de la discontinuité pneumatique de pression ou de dépression s'effectue de préférence par mesure du débit ou détection de la variation de celui-ci à l'aide d'un détecteur de débit 33, utilisé avec une source d'aspiration 34, par exemple dans un montage comme celui représenté sur la figure 7.

Ce détecteur est placé en série dans la ou les ligne(s) d'aspiration et de commande par variation pneumatique existant entre la pièce à main et le générateur de dépression.

Il fonctionne de façon classique à la manière d'un détecteur de débit. Le signal de détection engendré par la discontinuité de dépression est traité par un circuit électronique intégré au détecteur 33 et associé à l'unité centrale 35 du ou des appareil(s) commandé(s). L'unité centrale 35 peut par exemple commander un seul appareil ou la ou l'une des voies par exemple d'un appareil de génération d'un jet liquide pulsé-aspiré délivré par la pièce à main ou tout autre appareil alimentant la pièce à main, tel que par exemple un appareil de distribution du liquide de travail 36 et un appareil générateur de l'aspiration 37. L'unité centrale 35 peut en outre commander tout autre appareil lié à l'intervention.

Pour des raisons de sécurité le signal du détecteur n'est pris en compte qu'à partir d'un certain seuil. On évite ainsi les fausses détections liées au passage fortuit de la main de l'opérateur, d'un pan de manche ou tout autre perturbation dans le champ d'aspiration de l'orifice de commande existant sur le corps de la pièce à main.

Pour augmenter encore la sécurité, on peut chercher à augmenter la faible constante de temps ou le retard existant naturellement entre l'action manuelle de commande et sa détection, liée à l'inertie de la chaîne de détection.

Pour ce faire une solution intéressante consiste à limiter le débit en remplaçant, entièrement ou en partie seulement, le conduit pneumatique 38 reliant le détecteur 33 au générateur 34 de dépression ou de pression par un tronçon 39 de plus faible diamètre. Pour des raisons de commodité et de garantie de stérilité, on pourra préférer remplacer ce conduit 38 dans son intégralité par un conduit de plus faible diamètre par exemple entre le détecteur et le générateur de dépression ou de pression.

Le débit étant plus faible en raison de la moindre section du conduit, la constante de temps jusqu'à la détection est augmentée. Une action fortuite, fugitive et brève devant un orifice d'aspiration et de commande sur la pièce à main restera sans effet sur la commande.

La figure 8 a pour but de montrer le détecteur 33 tel qu'utilisé dans l'exemple de montage représenté sur la figure 7 et pouvant être utilisé dans la pratique.

On décrira maintenant le détecteur choisi sans pour autant considérer qu'il s'agit du seul possible.

Le détecteur représenté sur la figure 8 est formé à partir d'un capteur de débit d'air fabriqué et vendu par la société HONEYWELL sous la référence AVM 300. Le détecteur se compose d'une branche parallèle 40 de diamètre réduit et calibré aux extrémités de laquelle est monté en dérivation le capteur de débit 41 de marque HONEYWELL. L'ensemble du détecteur est inséré en série dans le conduit 42 reliant l'orifice de commande sur la pièce à main au générateur d'aspiration ou de pression 34. La sortie du détecteur passe entre zéro de tension dans un état de non-détection à une tension maximale par exemple de 5 volts lors de la détection et revient ensuite à un niveau zéro lors du retour à l'état initial. Ce signal de saut de tension est exploité pour servir de commande à un appareil lié à l'utilisation de la pièce à main, par exemple à son alimentation en fluide de travail et éventuellement à l'aspiration. Une succession d'actions digitales effectuées par l'utilisateur se traduit par un train d'impulsions en sortie qui sera convenablement exploité pour servir de commande.

L'invention se rapporte également au procédé de commande d'un appareil lié à l'intervention réalisée à partir de cette pièce à main selon lequel l'opérateur forme une ou plusieurs discontinuité(s) de pression ou de dépression par une action digitale sur une zone, une conformation spécifique ou une touche sur le corps de cette pièce à main reliée par un conduit à un générateur de pression ou de dépression, et selon lequel on détecte cette ou ces discontinuité(s) par un détecteur équipé d'un capteur associé à un circuit générant un signal ou des signaux de détection, que l'on exploite celui-ci ou ceux-ci pour la commande de fonctionnement de ou des appareil(s) lié(s) à l'intervention réalisée avec cette pièce à main constituant ainsi une véritable commande à distance pour le ou les appareil(s) visé(s).

Selon le procédé de commande la pluralité des discontinuités de pression ou de dépression est formée d'une succession d'actions digitales pratiquées par l'opérateur sur la pièce à main, détectées par le détecteur et transformées en signaux de détection et interprétées par un circuit de décodage en vue de la commande à distance de ou des appareil(s) visé(s).

## Revendications

1. Ensemble de commande de fonctionnement pour pièce à main à usage chirurgical ou médical présentant sur le corps de la pièce à main au moins une commande manuelle de fonctionnement **caractérisé en ce qu'**il se compose :
. d'une pièce à main comprenant un conduit de commande,
. du conduit de commande de la pièce à main celui-ci transportant le fluide gazeux de commande et reliant la pièce à main à un générateur (34) de pression ou de dépression,
. d'un circuit de détection qui transforme les discontinuités de pression ou de dépression provenant d'une action digitale de l'opérateur en signaux électriques exploitables pour la commande à distance d'un appareil ou d'une unité fonctionnelle en relation avec le travail médical ou chirurgical de l'opérateur utilisant la pièce à main pour son travail,
et **en ce que** le conduit de commande est relié au générateur (34) à travers le circuit de détection qui est configuré pour exploiter le signal électrique de commande en vue de réaliser une ou plusieurs commandes de fonctionnement de l'appareil ou de l'unité fonctionnelle en relation avec le travail médical ou chirurgical de l'opérateur utilisant la pièce à main pour son travail.

2. Ensemble de commande de fonctionnement pour pièce à main selon la revendication 1 **caractérisé en ce que** la pièce à main utilise un liquide stérile de travail en haute pression et/ou en débit et est composée d'un corps (2) et d'une tête (3) et traversée de part en part par un conduit (4) de jet haute pression ou un conduit (5) de débit débouchant par la tête (3) de la pièce à main (1), la pièce à main étant reliée à un générateur de pression de liquide alimentant la pièce à main en liquide de travail.

3. Ensemble de commande de fonctionnement pour pièce à main selon la revendication précédente **caractérisé en ce que** la pièce à main est traversée par un conduit d'aspiration relié à une source ou à un générateur d'aspiration.

4. Ensemble de commande de fonctionnement pour pièce à main selon la revendication 1 **caractérisé en ce que** les signaux de commande sont des discontinuités de dépression ou d'aspiration engendrées par une action manuelle de l'opérateur sur la commande spécifique prévue sur la pièce à main.

5. Ensemble de commande de fonctionnement pour pièce à main selon la revendication 1 **caractérisé en ce que** les signaux de commande sont des discontinuités de débit.

6. Ensemble de commande de fonctionnement pour pièce à main selon la revendication 1 **caractérisé en ce que** les commandes de fonctionnement du ou des appareil(s) alimentant la pièce à main sont des discontinuités de dépression d'au moins un conduit de commande (11) ou (12) relié à une source de pression ou à une source de dépression.

7. Ensemble de commande de fonctionnement pour pièce à main selon la revendication précédente **caractérisé en ce que** les commandes de fonctionnement du ou des appareil(s) alimentant la pièce à main sont des discontinuités de dépression d'au moins un conduit de commande (11) ou (12) relié au réseau du vide d'un bloc opératoire.

8. Ensemble de commande de fonctionnement pour pièce à main selon la revendication 4 **caractérisé en ce que** les commandes de fonctionnement du ou des appareil(s) alimentant la pièce à main sont des discontinuités de pression d'au moins un conduit de commande (11) ou (12) relié à un compresseur.

9. Ensemble de commande de fonctionnement pour pièce à main selon l'une des revendications précédentes **caractérisé en ce qu'**au moins un conduit de commande (11) ou (12) est ouvert en permanence et est fermé momentanément pour la commande.

10. Ensemble de commande de fonctionnement pour pièce à main selon l'une des revendications précédentes de 1 à 8 **caractérisé en ce qu'**au moins un conduit de commande (11) ou (12) est fermé en permanence et est ouvert momentanément pour la commande.

11. Ensemble de commande de fonctionnement pour pièce à main selon la revendication précédente **caractérisé en ce qu'**au moins un conduit de commande (11) ou (12) est fermé en permanence par un clapet obturant l'orifice et actionné momentanément en ouverture par une action manuelle de commande.

12. Ensemble de commande de fonctionnement pour pièce à main selon la revendication 8 **caractérisé par** au moins un conduit de commande (11) ou (12) relié à la source de pression ou de dépression qui débouche sur le flanc de la pièce à main à proximité ou au niveau de la position naturelle d'au moins un doigt de l'utilisateur qui obture momentanément l'orifice du conduit adjacent directement avec son doigt ou par l'intermédiaire d'une pièce d'obturation.

13. Ensemble de commande de fonctionnement pour pièce à main selon l'une des revendications précédentes de 1 à 8 **caractérisé en ce qu'**au moins un conduit de commande (11) ou (12) présente un tronçon passant en saillie ou à découvert sur une zone de commande manuelle pouvant être couverte par un doigt de l'utilisateur pour une action digitale directe ou indirecte d'écrasement de la part de l'utilisateur.

14. Ensemble de commande de fonctionnement pour pièce à main selon la revendication 12 **caractérisé en ce que** la pièce intermédiaire est un bouton poussoir à rappel élastique vers le haut.

15. Ensemble de commande de fonctionnement selon la revendication précédente **caractérisé en ce que** la pièce intermédiaire présente une tête d'obturation en rappel élastique de dégagement venant en position opérante se plaquer contre l'orifice du conduit de commande, cette tête étant prolongée par un corps déformable traversé par l'air venant se rétracter avec étanchéité à l'air en position opérante de la tête d'obturation.

16. Ensemble de commande de fonctionnement pour pièce à main selon la revendication 1 **caractérisé en ce que** la pièce à main (1) comprend deux conduits de commande (11) et (12) traversant la pièce à main de part et d'autre du tube (4) ou (5) de distribution de liquide stérile et débouchant symétriquement chacun par un orifice sur les côtés du corps (2) de la pièce à main (1) au niveau de conformations ergonomiques et anatomiques (13) et (14) situées à proximité de l'emplacement du pouce et de l'index de l'opérateur maniant la pièce à main, les conduits de commande (11) et (12) étant reliés à un réseau de vide via des capteurs ou des détecteurs (33) sensibles à une variation de pression, de dépression ou de débit.

17. Ensemble de commande de fonctionnement pour pièce à main selon l'une quelconque des revendications 1 à 9 **caractérisé en ce que** les discontinuités de pression ou de dépression ou de débit sont détectées par un détecteur (33) ou modifient un capteur monté en série dans le conduit de liaison entre l'orifice de commande de la pièce à main et le générateur (34) pour être transmises en tant que commandes à l'unité centrale de fonctionnement (35) de l'appareil de fourniture du liquide stérile de travail à la pièce à main.

18. Ensemble de commande de fonctionnement pour pièce à main selon l'une quelconque des revendications précédentes **caractérisé en ce que** le détecteur de l'action d'obturation est un détecteur de débit (33) monté en série dans le conduit reliant la pièce à main (1) au générateur (34) de dépression ou de pression.

19. Ensemble de commande de fonctionnement pour pièce à main selon la revendication 17 **caractérisé en ce que** le conduit (38) ou une partie (39) de celui-ci entre le détecteur (33) et le générateur (34) de dépression ou de pression est de diamètre réduit.

20. Ensemble de commande de fonctionnement pour pièce à main selon l'une quelconque des revendications précédentes **caractérisée en ce que** la discontinuité de pression ou de dépression provoquée par l'opérateur est utilisée après détection et après exploitation pour réaliser une ou plusieurs commandes de fonctionnement d'au moins un appareil (36, 37) lié à la pièce à main ou différent de celle-ci mais intervenant dans l'opération générale.

21. Procédé de commande à partir d'un ensemble de commande de fonctionnement selon l'une quelconque des revendications précédentes **caractérisé en ce que** l'opérateur forme une ou plusieurs discontinuité(s) de pression ou de dépression par une action digitale sur une zone, une conformation spécifique ou une touche sur le corps de la pièce à main reliée par un conduit de commande (42) à un générateur (34) de pression ou de dépression indépendant du ou des fluide(s) de travail, **en ce que** l'on détecte cette ou ces discontinuité(s) par un détecteur (33) ou un capteur associé à un circuit générant un signal ou des signaux de détection, que l'on exploite celui-ci ou ceux-ci pour la commande de fonctionnement de l'appareil lié à l'intervention réalisée avec cette pièce à main constituant ainsi une véritable commande à distance pour l'appareil visé.

22. Procédé de commande selon la revendication 21 **caractérisé en ce que** la pluralité des discontinuités de pression ou de dépression est formée d'une succession d'actions digitales pratiquées par l'opérateur sur la pièce à main, détectées par le détecteur (33) puis transformées en signaux de détection et interprétées par un circuit de décodage en vue de la commande à distance de l'appareil visé.

## Patentansprüche

1. Funktionssteuerungsanordnung für ein Handstück zur chirurgischen oder medizinischen Verwendung, die an dem Körper des Handstücks wenigstens eine manuelle Funktionssteuerungseinrichtung aufweist, **dadurch gekennzeichnet,**
**dass** sich die Anordnung zusammensetzt aus:
- einem Handstück, das eine Steuerungsleitung aufweist,
- der Steuerungsleitung des Handstücks, die ein gasförmiges Steuerungsfluid transportiert und die das Handstück mit einem Druck- oder Druckabfall-Erzeuger (34) verbindet,
- einem Detektor-Schaltkreis, der die Diskontinuitäten des Drucks oder des Druckabfalls, die durch eine Fingereinwirkung der Bedienperson hervorgerufen sind, in für die Fernsteuerung eines Geräts oder einer Funktionseinheit in Zusammenhang mit der medizinischen oder chirurgischen Tätigkeit der das Handstück für ihre Arbeit verwendenden Bedienperson nutzbare elektrische Signale umwandelt,
und **dass** die Steuerungsleitung mit dem Erzeuger (34) über den Detektor-Schaltkreis verbunden ist, der zum Nutzen des elektrischen Steuersignals für die Bereitstellung einer oder mehrerer Funktionssteuerungen des Geräts oder der Funktionseinheit ausgebildet ist, das bzw. die in Zusammenhang mit der medizinischen oder chirurgischen Tätigkeit der Bedienperson steht, die das Handstück für ihre Arbeit verwendet.

2. Anordnung für ein Handstück nach Anspruch 1, **dadurch gekennzeichnet, dass** das Handstück eine sterile Hochdruck- und/oder Durchfluss-Arbeitsflüssigkeit verwendet und dass das Handstück aus einem Körper (2) und einem Kopf (3) gebildet und von einem seiner Enden bis zum anderen von einer Hochdruckstrahl-Leitung (4) oder einer Durchflussleitung (5) durchsetzt ist, die am Kopf (3) des Handstücks (1) mündet, wobei das Handstück mit einem Druckerzeuger für die Flüssigkeit verbunden ist, der das Handstück mit Arbeitsflüssigkeit versorgt.

3. Anordnung für ein Handstück nach Anspruch 2, **dadurch gekennzeichnet, dass** das Handstück von einer Absaugleitung durchsetzt ist, die mit einer Quelle oder einer Absaugeinrichtung verbunden ist.

4. Anordnung für ein Handstück nach Anspruch 1, **dadurch gekennzeichnet, dass** die Steuerungssignale Druckabfall- oder Absaug-Diskontinuitäten sind, die durch eine manuelle Einwirkung der Bedienperson auf die spezifische, am Handstück vorgesehene Steuerungseinrichtung hervorgerufen sind.

5. Anordnung für ein Handstück nach Anspruch 1, **dadurch gekennzeichnet, dass** die Steuerungssignale Durchfluss-Diskontinuitäten sind.

6. Anordnung für ein Handstück nach Anspruch 1, **dadurch gekennzeichnet, dass** die Funktions-Steuerbefehle des bzw. der Geräts bzw. Geräte, die das Handstück versorgen, Druckabfalls-Diskontinuitäten wenigstens einer Steuerungsleitung (11 oder 12) sind, die mit einer Druckquelle oder einer Druckabfall-Quelle verbunden ist.

7. Anordnung für ein Handstück nach Anspruch 6, **dadurch gekennzeichnet, dass** die Funktions-Steuerbefehle des oder der Geräts bzw. Geräte, die das Handstück versorgen, Druckabfalls-Diskontinuitäten wenigstens einer Steuerungsleitung (11 oder 12) sind, die an das Vakuumnetz eines Operationsblocks angeschlossen ist.

8. Anordnung für ein Handstück nach Anspruch 4, **dadurch gekennzeichnet, dass** die Funktions-Steuerbefehle des oder der Geräts bzw. Geräte, die das Handstück versorgen, Druck-Diskontinuitäten wenigstens einer Steuerungsleitung (11 oder 12) sind, die mit einem Kompressor verbunden ist.

9. Anordnung für ein Handstück nach einem der vorangehenden Ansprüche, **dadurch gekennzeichnet, dass** wenigstens eine Steuerungsleitung (11 oder 12) dauerhaft offen und zum Steuern zeitweise geschlossen ist.

10. Anordnung für ein Handstück nach einem der Ansprüche 1 bis 8, **dadurch gekennzeichnet, dass** wenigstens eine Steuerungsleitung (11 oder 12) dauerhaft geschlossen und zum Steuern zeitweise geöffnet ist.

11. Anordnung für ein Handstück nach dem vorangehenden Anspruch, **dadurch gekennzeichnet, dass** wenigstens eine Steuerungsleitung (11 oder 12) dauerhaft durch ein zum Verschließen der Öffnung ausgebildetes Ventil verschlossen ist, das zeitweise zum Öffnen durch eine manuelle Steuerungseinwirkung betätigbar ist.

12. Anordnung für ein Handstück nach Anspruch 8, **gekennzeichnet durch** wenigstens eine Steuerungsleitung (11 oder 12), die mit einer Druck- oder Druckabfall-Quelle verbunden ist und die an der Seite des Handstücks in der Nähe oder auf Höhe der natürlichen Position wenigstens eines Fingers der Bedienperson mündet, die die Öffnung der benachbarten Leitung zeitweise direkt mit ihrem Finger oder über ein Verschlussstück verschließt.

13. Anordnung für ein Handstück nach einem der Ansprüche 1 bis 8, **dadurch gekennzeichnet, dass** wenigstens eine Steuerungsleitung (11 oder 12) einen Abschnitt aufweist, der vorspringend oder offen in einem Bereich manueller Steuerung verläuft und der durch einen Finger der Bedienperson abgedeckt werden kann, um eine direkte oder indirekte Fingereinwirkung zum Zusammendrücken durch die Bedienperson zu ermöglichen.

14. Anordnung für ein Handstück nach Anspruch 12, **dadurch gekennzeichnet, dass** das Zwischenstück ein Druckknopf mit nach oben wirkender elastischer Rückstellkraft ist.

15. Anordnung nach Anspruch 11, **dadurch gekennzeichnet, dass** das Zwischenstück ein Verschlusskopf mit elastischer Freisetz-Rückstellkraft ist, der in Betriebsstellung gegen die Öffnung der Steuerungsleitung in Anlage tritt, wobei der Kopf durch einen deformierbaren und von Luft durchströmten Körper verlängert ist, der sich in der Wirkstellung des Verschlusskopfes luftdicht zusammenzieht.

16. Anordnung für ein Handstück nach Anspruch 1, **dadurch gekennzeichnet, dass** das Handstück (1) zwei Steuerungsleitungen (11) und (12) aufweist, die das Handstück beiderseits der Leitung (4) oder (5) zum Verteilen der sterilen Flüssigkeit durchziehen und die jeweils symmetrisch mit einer Öffnung auf den Seiten des Körpers (2) des Handstücks (1) auf Höhe von ergonomischen und anatomischen Ausformungen (13 und 14) münden, die benachbart der Lage des Daumens und des Zeigefingers der Bedienperson angeordnet sind, die das Handstück handhabt, wobei die Steuerungsleitungen (11 und 12) über für eine Veränderung des Drucks, des Druckabfalls oder des Durchflusses empfindliche Sensoren oder Detektoren (33) an ein Vakuumnetz angeschlossen sind.

17. Anordnung für ein Handstück nach einem der Ansprüche 1 bis 9, **dadurch gekennzeichnet, dass** die Druck- oder Druckabfall- oder Durchfluss-Diskontinuitäten durch einen Detektor (33) nachgewiesen werden oder auf einen Sensor einwirken, der zwischen die Verbindungsleitung zwischen der Steuerungsöffnung des Handstücks und den Erzeuger (34) geschaltet ist, um als Steuerbefehle an die zentrale Betriebseinheit (35) des Bereitstellungsgeräts für die sterile Arbeitsflüssigkeit des Handstücks übertragen zu werden.

18. Anordnung für ein Handstück nach einem der vorangehenden Ansprüche, **dadurch gekennzeichnet, dass** der Detektor für die Verschlusseinwirkung ein Durchflussdetektor (33) ist, der in die das Handstück (1) mit dem Druckabfall- oder Druck-Erzeuger (34) verbindende Leitung geschaltet ist.

19. Anordnung für ein Handstück nach Anspruch 17, **dadurch gekennzeichnet, dass** die Leitung (38) oder ein Abschnitt (39) derselben zwischen dem Detektor (33) und dem Druckabfall- oder Druck-Erzeuger (34) einen verringerten Durchmesser aufweist.

20. Anordnung für ein Handstück nach einem der vorangehenden Ansprüche, **dadurch gekennzeichnet, dass** die durch die Bedienperson hervorgerufene Druck- oder Druckabfall-Diskontinuität nach dem Nachweis und nach der Nutzung zum Realisieren einer oder mehrerer Funktionssteuerungen für wenigstens ein Gerät (36, 37) verwendet wird, das mit dem Handstück zusammenwirkt oder von diesem verschieden ist, jedoch in dem generellen Arbeitsvorgang zum Einsatz kommt.

21. Steuerungsverfahren unter Verwendung einer Funktionssteuerungsanordnung nach einem der vorangehenden Ansprüche, **dadurch gekennzeichnet, dass** die Bedienperson eine Druck- oder Druckabfall-Diskontinuität oder mehrere solcher Diskontinuitäten durch eine Fingereinwirkung auf einen Bereich, eine spezifische Ausformung oder eine Taste am Körper des Handstücks erzeugt, die über eine Steuerungsleitung (42) mit einem Druck- oder Druckabfall-Erzeuger (34) verbunden ist, der von dem Arbeitsfluid oder den Arbeitsfluiden unabhängig ist, und dass die Diskontinuität oder die Diskontinuitäten mittels eines Detektors (33) oder eines Sensors nachgewiesen werden, der einem ein Nachweissignal oder Nachweissignale erzeugenden Schaltkreis zugeordnet ist, und dass das Signal oder die Signale zur Funktionssteuerung des Geräts genutzt werden, das mit dem unter Verwendung des Handstücks durchgeführten Eingriff in Zusammenhang steht, so dass auf diese Weise eine echte Fernsteuerung für das betreffende Gerät geschaffen wird.

22. Verfahren nach Anspruch 21, **dadurch gekennzeichnet, dass** die Mehrzahl an Druck- oder Druckabfalls-Diskontinuitäten durch eine Abfolge von Fingereinwirkungen erzeugt wird, die die Bedienperson am Handstück vornimmt, die durch den Detektor (32) nachgewiesen werden und die anschließend in Nachweissignale umgewandelt und durch einen Dekodierungs-Schaltkreis für die Fernsteuerung des betreffenden Geräts interpretiert werden.

## Claims

1. Operating control assembly for handpiece for surgical or medical use, with at least one manual operating control on the handpiece body, **characterised in that** it comprises:
- a handpiece with a control pipe,
- the handpiece control pipe, transporting the gaseous control fluid and linking the handpiece to a pressure or vacuum generator (34),
- a detection circuit which transforms the pressure or vacuum discontinuities arising from a digital action of the operator into electrical signals which can be used for the remote control of an appliance or functional unit related to the medical or surgical work of the operator using the handpiece for his work,
and **in that** the control pipe is linked to the generator (34) through the detection circuit which is configured to use the electrical control signal with a view to implementing one or more operating commands of the appliance or functional unit related to the medical or surgical work of the operator using the handpiece for his work.

2. Operating control assembly for handpiece according to claim 1, **characterised in that** the handpiece uses a sterile working fluid at high pressure and/or flow and comprises a body (2) and a head (3), and through which passes a high pressure jet pipe (4) or a flow pipe (5) opening at the head (3) of the handpiece (1), the handpiece being linked to a fluid pressure generator supplying the handpiece with working fluid.

3. Operating control assembly for handpiece according to the previous claim, **characterised in that** through the handpiece passes a suction pipe linked to a suction source or generator.

4. Operating control assembly for handpiece according to claim 1, **characterised in that** the control signals are vacuum or suction discontinuities generated by a manual action of the operator on the specific control provided on the handpiece.

5. Operating control assembly for handpiece according to claim 1, **characterised in that** the control signals are flow discontinuities.

6. Operating control assembly for handpiece according to claim 1, **characterised in that** the operating controls of the appliance(s) feeding the handpiece are vacuum discontinuities from at least one control pipe (11) or (12) linked to a pressure or vacuum source.

7. Operating control assembly for handpiece according to the previous claim, **characterised in that** the operating controls of the appliance(s) feeding the handpiece are vacuum discontinuities from at least one control pipe (11) or (12) linked to the vacuum network of an operating block.

8. Operating control assembly for handpiece according to claim 4, **characterised in that** the operating controls of the appliance(s) feeding the handpiece are pressure discontinuities from at least one control pipe (11) or (12) linked to a compressor.

9. Operating control assembly for handpiece according to any of the previous claims, **characterised in that** at least one control pipe (11) or (12) is permanently open and closed momentarily for the control.

10. Operating control assembly for handpiece according to any of the previous claims 1 to 8, **characterised in that** at least one control pipe (11) or (12) is permanently closed and opened momentarily for the control.

11. Operating control assembly for handpiece according to the previous claim, **characterised in that** at least one control pipe (11) or (12) is permanently closed by a valve sealing the orifice and actuated momentarily to open by a manual control action.

12. Operating control assembly for handpiece according to claim 8, **characterised by** at least one control pipe (11) or (12) linked to the pressure or vacuum source and opening on the side of the handpiece close to or at the natural position of at least one finger of the operator who momentarily seals the orifice of the adjacent pipe directly with his finger or by a sealing piece.

13. Operating control assembly for handpiece according to any of the previous claims 1 to 8, **characterised in that** at least one control pipe (11) or (12) has a protruding or uncovered section on a manual control zone which may be covered by a finger of the operator for direct or indirect digital crushing action by the operator.

14. Operating control assembly for handpiece according to claim 12, **characterised in that** the intermediate piece is a push button with resilient upward return.

15. Operating control assembly according to the previous claim, **characterised in that** the intermediate part has a sealing head with resilient release return that in the operating position comes to rest against the orifice of the control pipe, this head being extended by a deformable body with air passing through which retracts sealed against air in the operating position of the sealing head.

16. Operating control assembly for handpiece according to claim 1, **characterised in that** the handpiece (1) comprises two control pipes (11) and (12) passing through the handpiece from either side of the sterile fluid distribution tube (4) or (5) and each opening symmetrically in an orifice on the sides of the body (2) of the handpiece (1) at the ergonomic and anatomical shapings (13) and (14) situated at the position of the thumb and index finger of the operator using the handpiece, the control pipes (11) and (12) being linked to a vacuum network via sensors or detectors (33) sensitive to a variation in pressure, vacuum or flow.

17. Operating control assembly for handpiece according to any of claims 1 to 9, **characterised in that** the pressure or vacuum or flow discontinuities are detected via a detector (33) or modify a sensor mounted in series in the connecting pipe between the control orifice of the handpiece and the generator (34) in order to be transmitted as commands to the central operating unit (33) of the appliance supplying the sterile working fluid to the handpiece.

18. Operating control assembly for handpiece according to any of the previous claims, **characterised in that** the sealing action detector is a flow detector (33) mounted in series in the pipe connecting the handpiece (1) to the vacuum or pressure generator (34).

19. Operating control assembly for handpiece according to claim 17, **characterised in that** the pipe (38) or part (39) thereof between the detector (33) and the vacuum or pressure generator (34) is of reduced diameter.

20. Operating control assembly for handpiece according to any of the previous claims, **characterised in that** the pressure or vacuum discontinuity caused by the operator is used, after detection and after use, to implement one or more operating controls of at least one appliance (36, 37) linked to the handpiece or separate therefrom but required in the general operation.

21. Control process from an operating control assembly according to any of the previous claims, **characterised in that** the operator creates one or more pressure or vacuum discontinuities by digital action on a zone, a specific shaping or key on the handpiece body which is linked via a control pipe (42) to a pressure or vacuum generator (34) independent of the working fluid(s), **in that** the discontinuity or discontinuities is/are detected by a detector (33) or sensor associated with a circuit generating a detection signal or signals which is/are then used for control of operation of the appliance linked to the intervention performed with this handpiece, thus constituting a true remote control for the appliance concerned.

22. Control process according to claim 21, **characterised in that** the plurality of pressure or vacuum discontinuities is formed by a succession of digital actions performed by the operator on the handpiece, detected by the detector (33) then transformed into detection signals and interpreted by a decoding circuit for the purpose of remote control of the appliance concerned.
